# EUROPEAN PATENT APPLICATION

(11) **EP 4 603 835 A1**
(43) Date of publication of application: **20.08.2025**
(21) Application number: 22961592.7
(22) Date of filing: 13.10.2022
(51) Int. Cl.: G01N 33/48

(54) **SALIVA COLLECTION METHOD AND SALIVA COLLECTION MEMBER AND METHOD FOR DETERMINING ABSENCE/PRESENCE OF BLEEDING AND METHOD FOR DETERMINING PROGRESS STATE OF PERIODONTAL DISEASE**

(71) Applicant: San Ark Lab, Inc., Sendai-shi, Miyagi 980-0021 (JP); Multifunctional Protein Research Institute Co., Ltd., Sendai-shi, Miyagi 981-0904 (JP)
(72) Inventor: KOMINE, Kenichi, Sendai-shi, Miyagi 981-0905 (JP); KOMINE, Yumiko, Sendai-shi, Miyagi 981-0905 (JP)
(74) Representative: Dr. Weitzel & Partner
(86) International application number: PCT/JP2022/038170
(87) International publication number: WO 2024/079839

(57) **Abstract**

A saliva-collecting materials, which enable simple and non-invasive saliva collection, was found. It can be used without any processing. That is, determination of occult blood in saliva and measurement of inflammatory lactoferrin polypeptide concentration in saliva are performed only by centrifuging the saliva-collecting materials. With the above contents, a determination method, which enables pathological classification of periodontal disease in humans and animals, is provided.

Saliva is collected noninvasively by selecting and using a non-woven fabric, which can be quantitatively collected. After collecting the saliva, the non-woven fabric is set in a filter and centrifuged, and the blood components remaining on the non-woven fabric after centrifugation are visually observed to detect salivary occult blood. In addition, by measuring the I-Lf concentration in the liquid phase sample after centrifugation, it is possible to classify the periodontal disease according to the pathology.

## Description

### TECHNICAL FIELD

The present invention relates a saliva-collecting method, saliva-collecting materials, a method for determining presence or absence of bleeding, and a method for determining progress of periodontal disease.

### BACKGROUND ART

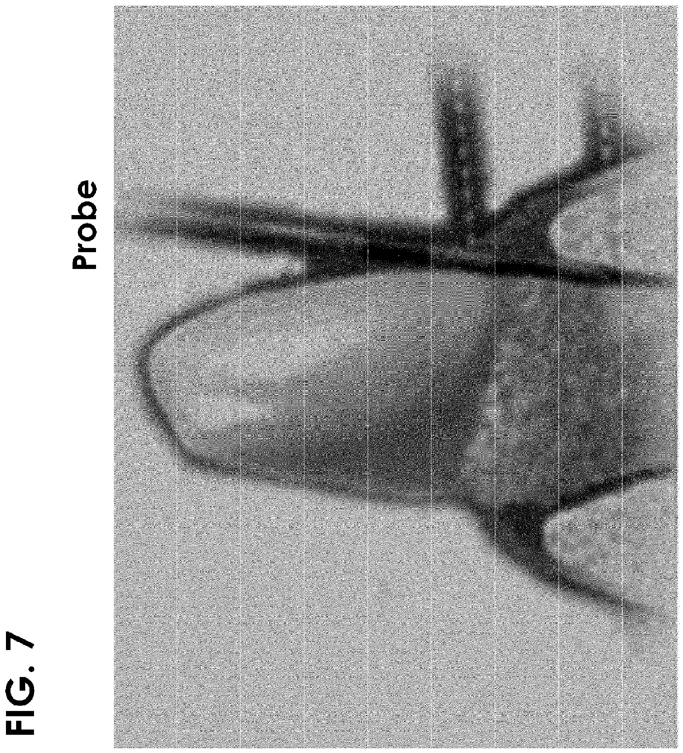

In humans and pets such as dogs, periodontal disease is a serious lifestyle-related disease that induces a wide variety of diseases such as endocarditis and diabetes (Non-Patent Document 1: Relationship between periodontal disease and lifestyle-related diseases, Edited by 8020 Promotion Foundation, 2005). Therefore, in humans, periodontal pocket measurement, bleeding, redness of periodontal tissue, etc. are examined using an instrument called a probe as shown in FIG. 7. However, examination of the affected area using the probe is often accompanied by pain.

In recent years, painless examinations, that is, non-invasive examinations such as measurement of occult blood in saliva associated with bleeding from periodontal tissue and measurement of blood components contained in saliva have been performed. However, there are problems such as the trouble of diluting at the time of measurement and the time required for measurement, and it cannot be said that it has spread yet.

On the other hand, in animals such as dogs, X-ray examinations and periodontal pocket measurements cannot be performed without general anesthesia. Therefore, there is a problem that the medical expenses are high because the burden on the pet itself is heavy and hospitalization is required (Non-Patent Document 2: Survey on medical fees for home-raised animals (dogs and cats) and survey on breeders' attitudes and survey results. Edited by Japan Veterinary Medical Association, 2015).

Cultivation of causative bacteria and genetic testing have also begun to be performed as tests using saliva, but a simple saliva test method that anyone can perform has not been established.

Periodontal disease is determined by observing whether the position of the periodontal tissue is lowered, by measuring the depth of the periodontal pocket with a probe, and by measuring the presence or absence of bleeding and pus due to periodontal disease. Determination is carried out at the dental site using determination methods such as OHI (Oral Hygiene Index) (Non-Patent Document 3: J. Amer. Dent Ass. 61, 172-179, 1960) and PLI (Plaque Index) (Non-Patent Document 4: Acta. Ddont. Scand. 22, 121-135, 1964) based on plaque index evaluation, and GI (Gingival Index) (Non-Patent Document 5: Acta. Odont. Scand. 21, 533-551, 1963), which evaluate gingival inflammation.

The above-mentioned method increases the cost of the examination and also places a heavy burden on the patient. Therefore, there is a demand for a non-invasive examination method that is effective and inexpensive for determining the condition of periodontal disease.

Furthermore, these determination methods are time-consuming determination methods because they are performed for all teeth, and are determination methods that require skill. In addition, a determination method using saliva is beginning to be introduced as a pain test for inflamed gingival tissue due to periodontal disease.

The following techniques have been introduced as non-invasive determination methods.

Saliva occult blood detection kit (Patent Document 1: JP 4590581 B2, Non-Patent Document 6: Nihon shishuubyou gakkai kaishi 43 (4), 416-423, 2001).

Fecal occult blood detection kit (Non-Patent Document 7: Research on saliva test standardization, 8020 Promotion Foundation Designated research project report, 2012).

Method for measuring lactate dehydrogenase (Patent Document 2: JP 2010-130924 A, Non-Patent Document 8: Nihon shishuubyou gakkai kaishi 44 (3), 261-272, 2002)

However, in the immunochromatographic saliva occult blood detection kit, 1 mL or more of saliva is expelled into a paper cup using sugar-free gum, etc., and diluted 5 times with water to suppress the influence of substances such as mucin that inhibit the development on the membrane, or the discharged liquid after rinsed with 3 mL of water for 10 seconds is required (Non-Patent Document 9: Document insert of Perioscreen "Sunstar", 2017).

On the other hand, in the salivary occult blood test using the fecal occult blood detection kit, the saliva is collected and measured in the same way as the immunochromatographic kit. But, it must be done by an external laboratory equipped with an expensive automated analyzer (Non-Patent Document 12: Document insert of fecal occult blood kit OC-Hemodia AutoIII 'EIKEN CHEMICAL CO., LTD.', 2021).

In addition, normal bacterial testing methods to detect and determine periodontal disease-causing bacteria contained in saliva and methods using the polymerase chain reaction (PCR) method, which is a genetic test, are also performed at external testing institutions equipped with dedicated equipment(Non-Patent Document 10: GCCIRCLE. 141, 2012-2016, 2012, Patent Document 3: JP 2017-85944 A).

Because subjective symptoms are less likely to appear in early periodontal disease, these examinations are intended for patients with moderate or higher patients, who have already experienced subjective symptoms such as bleeding when using a probe or beginning to worry about bad breath.

On the other hand, in animals such as dogs, periodontal disease is generally examined under general anesthesia (Non-Patent Document 13: Periodontal disease in dogs (diagnostic method) | POCKET PETS DOCTORS https://pocketpetsdoctors.com/?p=134, 2021).

We have already reported that the "inflammatory lactoferrin polypeptide (I-Lf)" appears in the saliva of patients with periodontal disease (Non-Patent Document 11: Mol. Immunol. 44, 1498-1508, 2007, Patent Document 4: JP 4029988 B2), and have shown by Enzyme-Linked Immuno Sorbent Assay (ELISA) method that the above is effective in detecting patients with early periodontal disease without using a probe.

Similar to the measurement of I-Lf, measurement of salivary occult blood, which determines bleeding in saliva, is performed as described above for early detection of periodontal disease. Among them, the measurement of salivary occult blood using a non-woven fabric has also been reported (Patent Document 6: JP 2860660 B2). After immersing the hemolytic component and various chemicals in the non-woven fabric, the non-woven fabric is dried and then attached to a support such as polyvinyl chloride to determine the chromaticity. More specifically, in Patent Document 6, an indicator, which is oxidized with a color change in the presence of a blood molecular group present in blood, an oxidizing agent effective for oxidizing the indicator, buffering agent to maintain pH in the range of 4-7, activators to increase sensitivity, and saponins are impregnated into absorbent carriers selected from paper, cellulose, synthetic fibers, synthetic resin woven fabric and non-woven fabric.

As the non-woven fabric, the non-woven fabric described in Patent Document 7 is known. This non-woven fabric has a weight of 30-80 g/m² according to JIS P8124:1998, a sheet density of 0.01-0.05 g/cm³, a compression work load of 0.85-5.0 gf cm/cm² according to the KES method, and a wet tensile strength of 8 N/100 mm or more. However, its use is cooking paper, and there is no disclosure of other uses.

### PRIOR ART DOCUMENTS

### PATENT DOCUMENTS

Patent Document 1: JP 4590581 B2
Patent Document 2: JP 2010-130924 A
Patent Document 3: JP 2017-85944 A
Patent Document 4: JP 4029988 B2
Patent Document 5: JP 4627263 B2
Patent Document 6: JP 2060660 B2
Patent Document 7: JP 6259365 B2

### NON-PATENT DOCUMENTS

Non-Patent Document 1: Relationship between periodontal disease and lifestyle-related diseases, Edited by 8020 Promotion Foundation, 2005
Non-Patent Document 2: Survey on medical fees for home-raised animals (dogs and cats) and survey on breeders' attitudes and survey results. Edited by Japan Veterinary Medical Association, 2015
Non-Patent Document 3: J. Amer. Dent Ass. 61, 172-179, 1960
Non-Patent Document 4: Acta. Odont. Scand. 22, 121-135, 1964
Non-Patent Document 5: Acta. Odont. Scand. 21, 533-551, 1963
Non-Patent Document 6: Nihon shishuubyou gakkai kaishi 43 (4), 416-423, 2001
Non-Patent Document 7: Research on saliva test standardization, 8020 Promotion Foundation Designated research project report, 2012
Non-Patent Document 8: Nihon shishuubyou gakkai kaishi 44 (3), 261-272, 2002
Non-Patent Document 9: Document insert of Perioscreen "Sunstar", 2017
Non-Patent Document 10: GCCIRCLE. 141, 2012-2016, 2012
Non-Patent Document 11: Mol. Immunol. 44, 1498-1508, 2007
Non-Patent Document 12: Document insert of fecal occult blood kit OC-Hemodia AutoIII 'EIKEN CHEMICAL CO., LTD.', 2021
Non-Patent Document 13: Periodontal disease in dogs (diagnostic method) | POCKET PETS DOCTORS https://pocketpetsdoctors.com/?p=134, 2021
Non-Patent Document 14: J. Clin. Pathol. 31, 139-143, 1978
Non-Patent Document 15: Clin. Chim. Acta. 136, 95-104, 1984

### DISCLOSURE OF THE INVENTION

### PROBLEMS TO BE SOLVED BY THE INVENTION

In the present invention, in order to quantify I-Lf in saliva, there are problems of selecting a non-woven fabric, from which saliva can be quantitatively collected, and establishing a measurement method. In addition, when collecting, it is necessary to use a non-invasive method, which does not cause pain to patients and infected animals such as pets. In particular, pets currently need to be given general anesthesia when observing the oral cavity, but there is a problem of establishing a collection method, which does not require anesthesia.

As a non-invasive saliva-collecting method, it is assumed to be used in a method of collecting saliva by inserting it in the oral cavity or by exhaling it. And, a non-woven fabric, which is safe to use on the living body and is being considered as saliva-collecting materials that can measure salivary occult blood, is selected. As the non-woven fabric, commonly available materials instead of a special processing method are used. For this reason, a method, which can easily and quickly determine salivary occult blood by using a non-woven fabric after saliva absorption, is investigated. The orthotolidine method and the guaiac method, which are chemical test methods, are used as routine tests for fecal occult blood, and are also used for some salivary occult blood tests.

In addition, immunological methods using antigen-antibody reaction are used for lower gastrointestinal tract bleeding, large intestine bleeding, and salivary occult blood (Non-Patent Document 9: Document insert of Perioscreen "Sunstar", 2017).

In Patent Document 6, a hemolytic component and various chemicals are immersed in the non-woven fabric, dried, and attached to a support such as polyvinyl chloride in order to be determined by the chromaticity. However, it requires complicated work and materials. In addition, although the details of the non-woven fabric are not disclosed, when a commercially available non-woven fabric is used, the amount of saliva necessary for determination cannot be absorbed, and therefore stable determination cannot be performed. The present inventors further investigated the cause of the inability to perform stable determination, and found that the cause lies in the variation in the amount of absorption. In the present invention, as the result of searching for a non-woven fabric capable of controlling the amount of absorption constant, it was found that the non-woven fabric described in Patent Document 7 has a large amount of saliva absorption and a small variation. The non-woven fabric is also used to filter highly viscous edible oils, etc., and has a high ability to retain contaminants. Therefore, the inventors have found that it is possible to detect occult blood donation in saliva by attaching a non-woven fabric that has absorbed saliva to a centrifugal sterilization filter or a centrifugal ultrafiltration filter.

Among currently reported measurement methods such as salivary occult blood measurement, I-Lf, and causative bacteria, no test method for classifying the pathology of periodontal disease from saliva samples has been established. Therefore, a judgment method, which enables periodontal disease classification using both I-Lf concentration and salivary occult blood that are effective for detecting periodontal disease and are also judgment indicators in saliva, will be established, and the problems will be solved.

An object of the present invention is to provide a saliva-collecting method, which can be performed in humans for easy periodontal disease examination and in animals such as dogs and cats without anesthesia.

An object of the present invention is to provide a saliva-collecting method and saliva-collecting materials, a method for determining presence or absence of bleeding, and a method for determining progress of periodontal disease. These provisions are simple and non-invasive, can be used without any processing, and can be used to determine occult blood in saliva and detect inflammatory lactoferrin polypeptide concentration in saliva only by centrifugation without the need for complicated work and materials.

An object of the present invention is to provide a method for easily and quickly determining the progress of periodontal disease by using saliva as a sample. In addition, a saliva-collecting method, which is non-invasive, allows people himself/herself to determine periodontal disease without pain from the probe, and allows pet owners to determine periodontal disease of the pet without the need for anesthesia, is provided.

### SOLUTIONS FOR SOLVE THE PROBLEMS

The present invention is a saliva-collecting method for periodontal disease examination, in which saliva-collecting materials made of the non-woven fabric capable of quantitatively absorbing saliva are made to absorb the saliva of humans or animals such as dogs and cats.

The non-woven fabric preferably has a basis weight of 30 to 80 g/m² according to JIS P2184:1998, a sheet density of 0.01 to 0.05 g/cm³, a compression work load of 0.85 to 5.0 gf · cm/cm² according to the KES method, and a wet tensile strength of 8 N/100 mm or more.

The present invention is a method for determining presence or absence of bleeding, in which after saliva is collected by the saliva-collecting method, the saliva-collecting materials containing saliva are attached to a centrifugal filtration filter and centrifuged, and the blood cell components retained in the saliva-collecting materials after centrifugation are observed.

The present invention is a method for determining the progress of periodontal disease by measuring both inflammatory lactoferrin polypeptide in the liquid phase obtained by the centrifugation and occult blood measurement.

The present invention is saliva-collecting materials used in the saliva-collecting method for periodontal disease examination, and made of the non-woven fabric capable of quantitatively absorbing saliva.

The non-woven fabric preferably has a basis weight of 30 to 80 g/m² according to JIS P2184:1998, a sheet density of 0.01 to 0.05 g/cm³, a compression work load of 0.85 to 5.0 gf · cm/cm² according to the KES method, and a wet tensile strength of 8 N/100 mm or more.

### EFFECTS OF THE INVENTION

The effects of the present invention will be described together with knowledge obtained in making the present invention.

The present inventors have found that the non-woven fabric described in Patent Document 7 has the following characteristics necessary for determining periodontal disease using I-Lf concentration and salivary occult blood as indices.
(1) Amount of saliva absorbed per unit volume is large.
(2) Variation in the amount of saliva absorbed per unit volume (and therefore variation in the amount of saliva absorbed as a whole) is extremely small.

Based on the discovery of such characteristics, the present invention was made based on the development of saliva-collecting materials for use in determining periodontal disease based on I-Lf concentration.

A non-woven fabric manufactured by Oji Kinocloth Co., Ltd. (product name "Reed") prepared by the air-laid method is used for collecting saliva. The non-woven fabric has a higher wet tensile strength than non-woven fabrics produced by other methods, is resistant to tearing even in physical treatments such as centrifugation, and has properties such that paper pieces are less likely to come off.

Measurements of I-Lf by immuno-nephelometry require 2-3 repeat measurements to increase confidence in the measurements. Therefore, 40 µL of sample volume is required for two measurements. Furthermore, in consideration of the case where the sample is diluted by immuno-nephelometry for re-measurement, it is necessary that the volume of the sample to be collected is 60 µL or more. In the present invention, the non-woven fabric is cut to a volume of 84 mm³, which can be attached to the reservoir of the centrifugal filtration filter, and it can be confirmed that 100 µL of saliva, which is highly viscous among body fluids, is quantitatively absorbed as 99.5 + 0.5 µL (Table 1).

**[Table 1]**

| A | | B | | C | |
|---|---|---|---|---|---|
| 84mm³ | 168mm³ | 84mm³ | 168mm³ | 84mm³ | 168mm³ |
| 100±0.5µL | 200±0.65µL | 40.2±64.2µL | 61.1±139.3µL | 35.3±65.6µL | 56.1±145.7µL |

| | | | | | |
|---|---|---|---|---|---|
| Amount of saliva added: 84mm³ 100µL, 168mm³ 200µL Number of repeated rests: 11 times Non-woven fabric of A Inc.: Oji Kinocloth Co., Ltd. Non-woven fabric of B Inc. Non-woven fabric of C Inc. Lower row: Absorbed amount (µL) ± Standard deviation (µL) | | | | | |

As shown in Patent Document 7, the non-woven fabric is made from wood pulp as raw materials. Also, natural fibers such as cotton and rayon, synthetic fibers such as polyester and polypropylene, and the like can be used as long as they have the same performance as the cooking paper, which can absorb highly viscous oil. Therefore, the non-woven fabric made of the above materials is adjusted according to the amount of saliva to be collected, and the necessary amount of saliva for examination can be collected simply and quickly in the non-invasive manner.

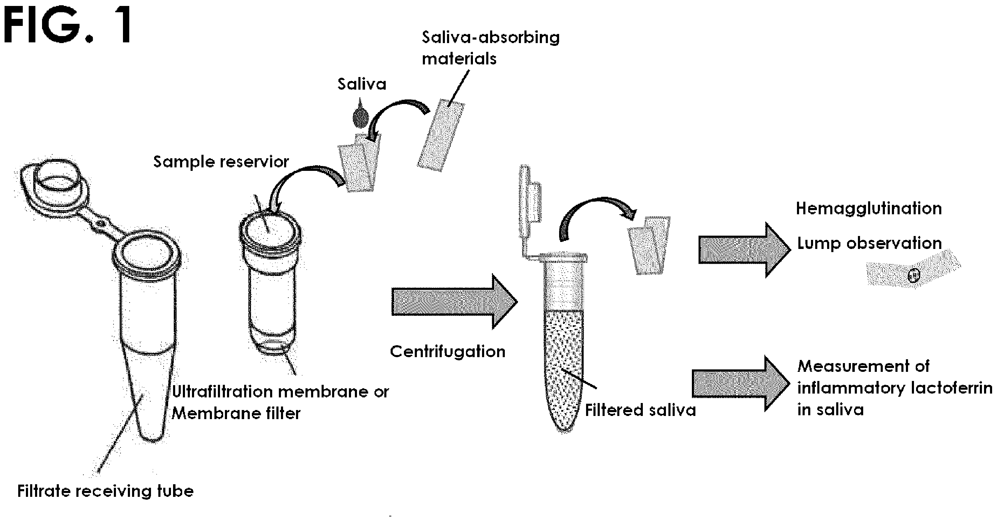

In addition, the collected saliva is set to a membrane filter with a pore size of 0.2 µm or 0.45 µm to eliminate the effects of bacteria, etc., a centrifugal filtration filter equipped with an ultrafiltration membrane that matches the molecular weight of the saliva component, which is a measurement index. , or a centrifugal filtration device with a similar function. Then, the saliva is centrifuged for about 5 to 15 minutes at 1,000 to 3,000 G (centrifugal force), which conforms to the standard of filtering instruments. I-Lf contained in the liquid phase after centrifugation and serving as a judgment index for periodontal disease can be measured by an antigen-antibody reaction, an enzyme reaction, a chemical color reaction, or the like. On the other hand, the occult blood in the saliva remaining in the saliva collecting materials after centrifugation could be visually checked for bleeding on the non-woven fabric. Until now, non-woven fabrics have required processing such as the addition of coloring agents. However, in the present invention, it was shown that it is a method that can be easily determined only by attaching to a sterilizing filter and centrifuging without requiring the above processing. The method of attaching the non-woven fabric to the centrifugal filtration device is shown in FIG. 1. By folding it in a V-shape or U-shape and attaching it to the device, blood cell components are accumulated in the non-woven fabric, which contacts the bottom of the filtration filter, making it possible to visually confirm it. The non-woven fabric used in the present invention differs from the non-woven fabric produced by another method in the amount of saliva absorbed by more than twice. In addition, it was found that the non-woven fabric made by other methods could not stably collect the necessary and sufficient amount of saliva samples for measurement because the variation in retention amount was more than 100 times different. Therefore, it was shown that the use of this non-woven fabric is an extremely effective saliva-collecting materials in the present measurement method.

In addition, in order to quantitatively confirm even a small amount of bleeding, the present invention established a method that the blood components contained in the non-woven fabric after centrifugation are dispersed in a buffer suitable for each measurement method, for example, the cyanmethemoglobin method, which is an international standard method, (Non-Patent Document 14: J. Clin. Pathol. 31, 139-143, 1978) or Triton/NaOH method (Non-Patent Document 15: Clin. Chim. Acta. 136, 95-104, 1984) etc.

According to the present invention, it is possible to provide a saliva-collecting method, which allows easy periodontal disease examination in humans, and can be easily conducted in animals such as dogs and cats without anesthesia.

According to the present invention, a saliva-collecting method and saliva-collecting materials, a method for determining presence or absence of bleeding, and a method for determining progress of periodontal disease can be provided. These provisions are simple and non-invasive, can be used without any processing, and can be used to determine occult blood in saliva and detect inflammatory lactoferrin polypeptide concentration in saliva only by centrifugation without the need for complicated work and materials.

Table 1 shows the results of an examination of the amount of saliva absorbed by the non-woven fabric.

### BRIEF DESCRIPTION OF THE DRAWINGS

FIG. 1 shows a processing method of saliva sample and a flow chart up to measurement.

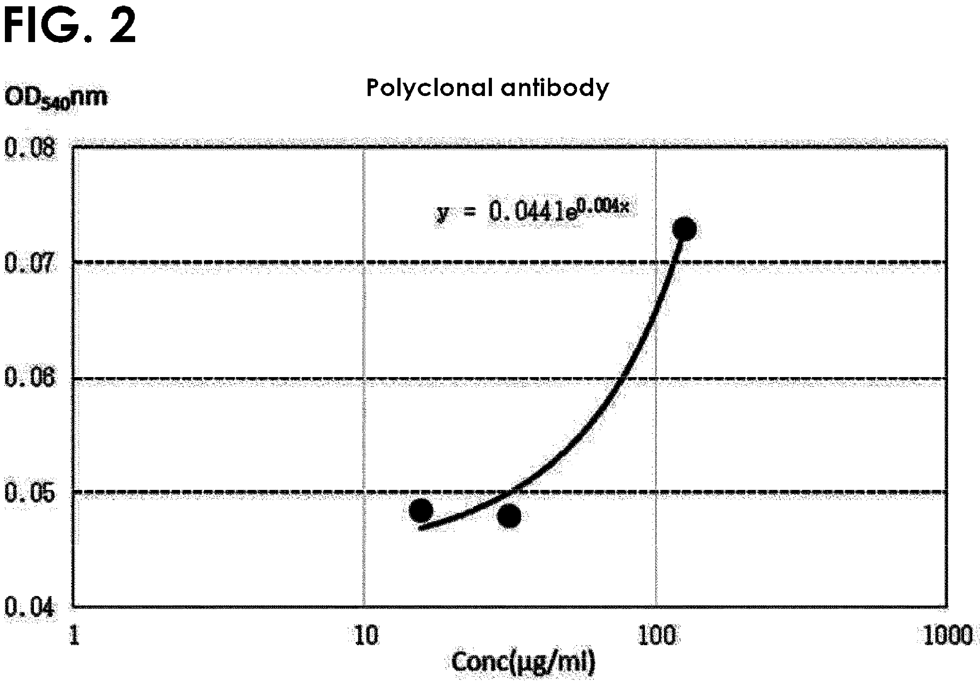

FIG. 2 shows an example of standard curve creation by immuno-nephelometry.

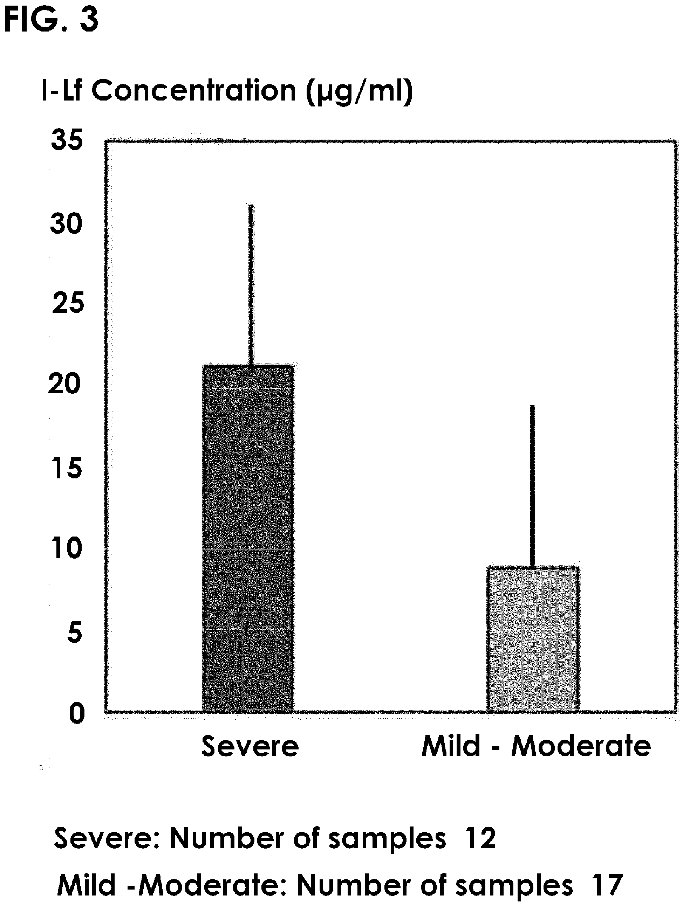

FIG. 3 shows an I-Lf concentration according to the disease state of periodontal disease infected animal (dog) saliva sample.

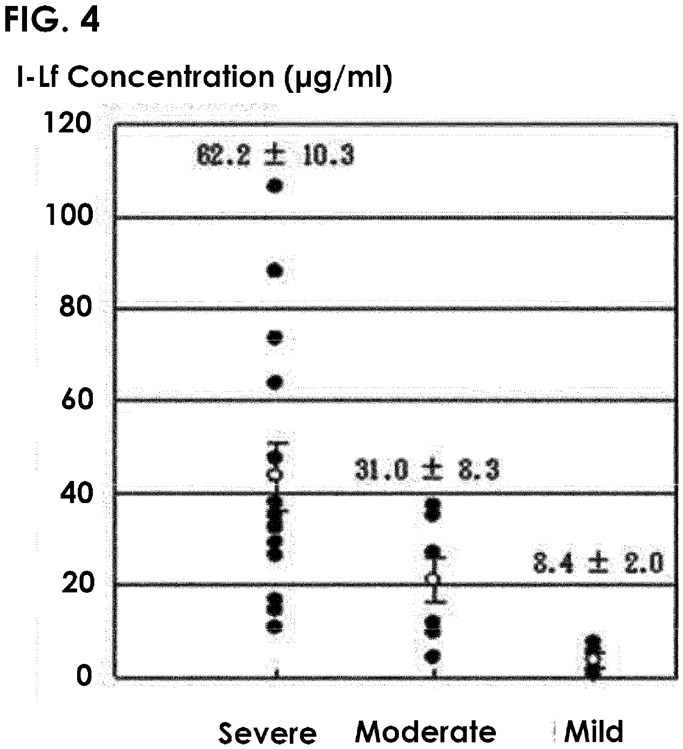

FIG. 4 shows an I-Lf concentration according to the medical condition of saliva samples from patients with periodontal disease.
FIG. 5 shows an occult blood observation sensitivity test in saliva using non-woven fabric used in the present invention.
FIG. 6 shows an appearance photograph showing a bleeding sample from a dog with periodontal disease.
FIG. 7 shows a schematic diagram of periodontal disease diagnosis using a probe.

### DESCRIPTION OF EMBODIMENTS

The present invention simultaneously measures the I-Lf, which is a very early periodontal disease determination marker contained in the saliva of humans and animals suffering from periodontal disease, and the presence or absence of bleeding, which increases in saliva due to bleeding associated with the progression of periodontal disease, And then, the progress of periodontal disease is determined.

In this test, the non-woven fabric (product name "Reed") of Oji Kinocloth Co., Ltd. was cut into a size of 84 mm³ in advance so that it could absorb 100 µL of human and dog saliva as saliva-absorbing materials, and the saliva was collected from patients and infected animals. The size is not limited to the size described above, and any size, which allows absorption of 100 µL of the sample, may be used. From that point, it is preferably 80 to 90 mm³, and more preferably 82 to 86 mm³.

This saliva-absorbed non-woven fabric was bent and placed in a commercially available centrifugal filtration filter loaded with a membrane filter with a pore size of 0.2 µm, and centrifuged at 2,000 G for 5 minutes. And, the non-woven fabric after centrifugation was placed on a white acrylic plate or the like, and the presence or absence of blood cell components was visually observed and determined.

Next, about filtrate after centrifugation, the I-Lf was measured by an immuno-nephelometry, which allows measurement in a short period of time, using an affinity antibody prepared from anti-I-Lf rabbit serum. For the measurement, Tris-HCl buffer (pH 7.2) containing 4.5% polyethylene glycol 8000 was used as the reaction buffer, and 120 *µ* L of the reaction buffer was dispensed into each well of a microplate (MICROLON 762070, greirer bio-one), and kept at 37 °C. Next, 1 mg/mL of I-Lf affinity antibody was adjusted with Tris-HCl buffer, 15 µL of the adjusted buffer was dispensed into each well, mixed with a plate mixer, 20 µL of saliva sample after centrifugation was added, mixed again, and reacted for 8 minutes in a constant temperature bath at 37 °C. After completion of the reaction, the plate mixer was used for the immune complex to be floated again, and the absorbance was measured at 540 nm to determine the I-Lf concentration in the saliva. In addition, when the occult blood was observed in the saliva and was considered to exceed the measurement range, the sample was diluted with the Tris-HCl buffer and measured again.

In the dog saliva, the I-Lf concentrations were 8.89±5.25 pg/ml for mild to moderate, while in the group judged to be severe they were higher value of 21.21±11.26 µg/ml. However, the measurement results in the severe and mild to moderate groups varied widely, and it was difficult to classify each symptom only by measuring I-Lf concentration. On the other hand, the measurement time was greatly shortened to 5 to 10 minutes from the start of measurement to obtaining the results, compared to about 2 hours, which is the measurement time of conventional Enzyme-Linked Immuno Sorbent Assay method (ELISA method).

Also, the I-Lf concentrations in human saliva samples were 62.2±10.3 µg/ml in the saliva of the severe patient judged by the GI value, but they were 31.0±8.3 µg/ml in the saliva of a moderate patient, and they were 8.4±2.0 µg/ml in the mild patient saliva. It became lower in patients with milder symptoms.

This result suggests that changes in the I-Lf concentrations due to periodontal disease in humans are more sharply fluctuated than in dogs, and that the classification is easier than in dogs. In humans, various self-management measures such as dentifrice, mouth rinse solution, and interdental brush are taken to maintain a hygienic environment in the oral cavity, which suppresses the deposition of dental plaque and tartar. Therefore, it is speculated that individual differences due to pathological conditions have decreased. On the other hand, in the case of dogs, there are differences in management methods such as dentifrice and awareness regarding the maintenance of the oral hygiene environment, and individual differences in the oral hygiene environment are large, and it was inferred that the I-Lf concentrations varied greatly.

In the measurement of occult blood in saliva, red blood cells, which are non-hemolytic components, have a relatively large particle size, whereas hemoglobin, which is a hemolytic component, is difficult to visually observe. Therefore, commercially available hemoglobin (SIGMA Inc., Cat. No. H7379-5G) was dissolved in a phosphate physiological buffer, and serially diluted solutions from 1 mg/mL to 0.01 pg/mL were used to examine the measurement sensitivity of hemoglobin. Hemoglobin solutions of each concentration were soaked in the non-woven fabric, centrifuged at 2,000 G for 15 minutes using a commercially available centrifugal filtration filter equipped with a 0.45 µm membrane filter, taken out after separating the liquid sample, and observed visually. As a result, it was possible to confirm visually from 1 µg/mL to 1 mg/mL in this measurement method. On the other hand, the measurement sensitivity of the human salivary occult blood detection kit by immunochromatography, which is already commercially available, is 2 µg / mL to 500 (less than) µg / mL (Non-Patent Document 9: Document insert of Perioscreen "Sunstar", 2017). It was suggested to be approximately the same as the method of the present invention.

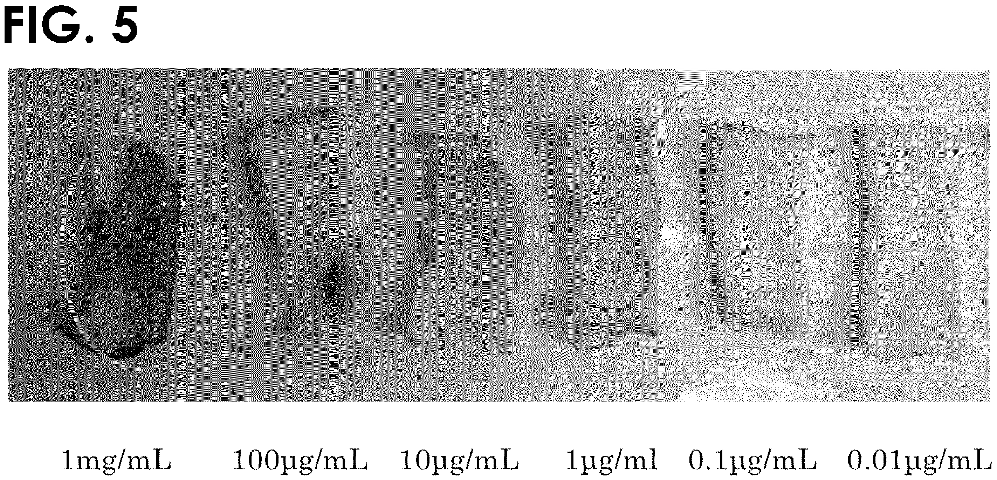

The results are shown in FIG. 5.

Based on the above results, observation of occult blood in saliva was performed on dog saliva. In this case, as the condition of periodontal disease worsened, the occult blood detection rate in saliva was not detected in samples with mild periodontal disease, but increased to 88% in moderate periodontal disease and 100% in severe periodontal disease. In addition, as the disease worsened, the sloughed epithelial tissue in the oral cavity was not detected in mild cases, but the detection rate increased to 12% in moderate cases and 80% in severe cases.

Observation of occult blood in saliva is effective for confirming the progress of periodontal disease, but it is difficult to detect mild periodontal disease only by observing occult blood. Therefore, it was suggested that the progress of periodontal disease can be determined more efficiently and accurately by combining with I-Lf concentration.

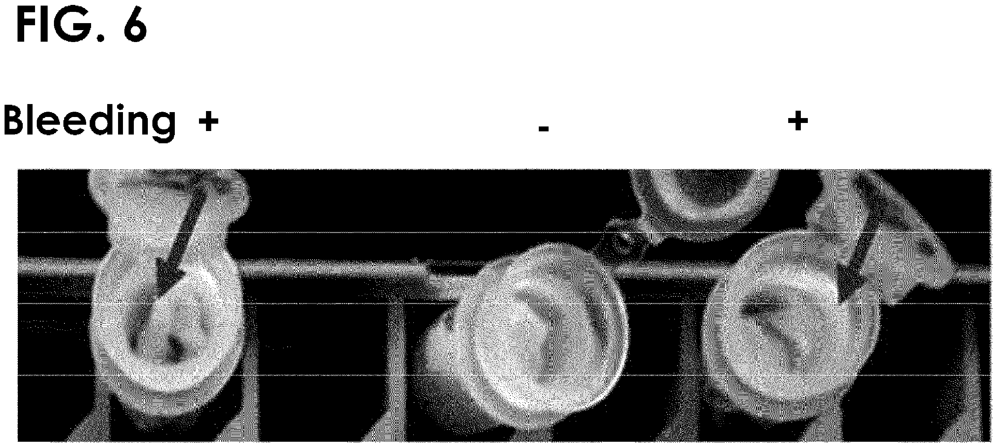

The results are shown in Table 2. That is, Table 2 shows the measurement results of salivary occult blood and sloughed epithelium of dogs suffering from periodontal disease. The bleeding sample is shown in FIG. 6.

**[Table 2]**

| | Bleeding | | Sloughed epithelial tissue | |
|---|---|---|---|---|
| | Absence | Presence | Absence | Presence |
| Mild cases | 100% | 0% | 100% | 0% |
| Moderate cases | 12% | 88% | 88% | 12% |
| Severe cases | 0% | 100% | 20% | 80% |

| | | | | |
|---|---|---|---|---|
| Mild cases: Number of samples 6 Moderate cases: Number of samples 11 Severe cases: Number of samples 12 | | | | |

Occult blood in saliva could be determined only by centrifuging the non-woven fabric without any processing. As a result, it was possible to simultaneously measure the two items of salivary occult blood and salivary I-Lf concentration, it made possible to determine the pathology of periodontal disease more accurately, and the determination time was dramatically shortened.

In addition, this method can be applied to the measurement of items other than I-Lf concentration, which are effective for judging periodontal disease. Because the non-woven fabric after saliva collection contains concentrated bacteria, which do not pass through the filtration membrane, it can be suspended in a small amount of sterile buffer and used as a concentrated sample for genetic testing and bacterial culture. In addition, the liquid phase portion can be used for various measurements such as immunoglobulins, other proteins, minerals, and enzymes. Therefore, it is a method, which can be applied in a wide range of fields such as primary screening for periodontal disease in general medical examinations of humans and animals, oral hygiene and health examinations.

Pets such as dogs require general anesthesia for detailed examinations, so periodontal disease tends to be more difficult to diagnose than in humans, and its discovery tends to be delayed. Therefore, it is often discovered after the disease becomes severe. But, in the present invention, since periodontal disease can be detected by the non-invasive examination method, early treatment can be started. Furthermore, it reduces the burden on pets due to general anesthesia and contributes to the reduction of expensive medical expenses for pets.

## Claims

1. A saliva-collecting method for periodontal disease examination, wherein saliva is collected by letting saliva-collecting materials comprising a non-woven fabric, which can absorb saliva quantitatively, absorb the saliva of humans or animals such as dogs and cats.

2. The saliva-collecting method according to claim 1, wherein the non-woven fabric has a basis weight of 30 to 80 g/m² according to JIS P2184:1998, a sheet density of 0.01 to 0.05 g/cm³, a compression work load of 0.85 to 5.0 gf · cm/cm² according to the KES method, and a wet tensile strength of 8 N/100 mm or more.

3. A method for determining presence or absence of bleeding, wherein saliva is collected by the saliva-collecting method according to claim 1, the saliva-collecting materials containing saliva are attached to a centrifugal filtration filter and centrifuged, and the blood cell component retained in the saliva-collecting materials after centrifugation are observed.

4. A method for determining progress of periodontal disease, wherein both inflammatory lactoferrin polypeptide in the liquid phase obtained by the centrifugation in claim 3 and occult blood measurement are measured.

5. Saliva-collecting materials used in a saliva-collecting method for periodontal disease examination, made of a non-woven fabric capable of quantitatively absorbing saliva.

6. The saliva collecting materials according to claim 5, wherein the non-woven fabric has a basis weight of 30 to 80 g/m² according to JIS P2184:1998, a sheet density of 0.01 to 0.05 g/cm³, a compression work load of 0.85 to 5.0 gf · cm/cm² according to the KES method, and a wet tensile strength of 8 N/100 mm or more.
